# EUROPEAN PATENT APPLICATION

(11) **EP 4 576 112 A1**
(43) Date of publication of application: **25.06.2025**
(21) Application number: 23217923.4
(22) Date of filing: 19.12.2023
(51) Int. Cl.: G16H 30/40, G16H 20/40, A61B 5/055, G01R 33/56, G06T 7/00, A61B 18/00

(54) **VISUALLY REPRESENTING POST-ABLATION LOCATIONS**

(71) Applicant: Koninklijke Philips N.V., 5656 AG Eindhoven (NL)
(72) Inventor: Smink, Jouke, 5656 AG Eindhoven (NL)
(74) Representative: Philips Intellectual Property & Standards

(57) **Abstract**

A mechanism for controlling a display of an anatomical structure. In particular, one or more visual properties of a post-ablation location on the anatomical structure are controlled responsive to ablation information (about an ablation performed at the post-ablation location) and MRI-derived information derived from one or more MR images of the post-ablation location.

## Description

### FIELD OF THE INVENTION

The present invention relates to the field of ablation procedures, and in particular to the display of information about an ablation procedure.

### BACKGROUND OF THE INVENTION

Cardiac arrhythmias are caused by an abnormal electrical (bio)circuit that makes the atria or ventricles beat quickly and flutter instead of fully squeezing. A known method to treat arrhythmias is catheter ablation, in which an ablation catheter is moved to an ablation location of an anatomical structure and controlled to perform ablation at the ablation location.

To improve an ease of performing a catheter ablation, it is common to make use of an electrical anatomical mapping system. Such a system typically uses a mapping catheter to measure one or more electrophysiological characteristics at different locations within the heart, and map the measured information on a 3D model of the heart. This 3D model may, for instance, have been generated by tracking the mapping catheter within the heart.

The electrophysiological characteristics may include, for instance, a voltage or activation time at a location. By interpolating measured electrophysiological characteristics about the 3D model, the electrical anatomical mapping system is able to create electrical anatomical maps, such as a voltage map or activation map.

When ablation is performed, it is also known to record the ablation locations. A display of the 3D model can be configured to identify the ablation locations. The display of each ablation location may be annotated with one or more ablation parameters, such as tip temperature, impedance, power and duration of the ablation.

There is an ongoing desire to increase the availability and ease of providing information about an ablation procedure to an operator, user or clinician.

### SUMMARY OF THE INVENTION

The invention is defined by the independent claims. Advantageous embodiments are defined in the dependent claims.

According to examples in accordance with an aspect of the invention, there is provided a computer-implemented method for controlling the visual representation of one or more post-ablation locations on a visual representation of an anatomical structure that has undergone one or more ablations.

The computer-implemented method comprises, for each post-ablation location: obtaining ablation information that comprises a value for each of one or more characteristics of a previous ablation that was performed on the anatomical structure at the post-ablation location; obtaining MRI-derived information derived from one or more magnetic resonance images of the post-ablation location; and controlling the visual representation of the post-ablation location on the visual representation of the anatomical structure responsive to both the ablation information and the MRI-derived information.

The present disclosure proposes to adjust or modify a visual representation of a post-ablation location responsive to one or more characteristics of the ablation (performed at the post-ablation location and one or more characteristics derived from MR imaging of the post-ablation location. This effectively provides a mechanism in which the visual properties of a visual representation (e.g., display) of a post-ablation location indicate information derived from two sources - a first source representing information of the ablation and a second source MR information about the post-ablation region.

Proposed approaches are based on the recognition that it would be advantageous to combine information about an ablation and (pre- and/or post-ablation derived) MRI information in a single visual representation. This provides a user or clinician with an increased amount of information for making clinical decisions at a same location, thereby reducing a risk of the user/clinician overlooking potentially important information or misunderstanding a relationship between two pieces of information associated with a same post-ablation location.

In some examples, for each post-ablation location, the MRI-derived information comprises, for each magnetic resonance image of the post-ablation location, at least one intensity value derived from one or more areas representing the post-ablation location within the magnetic resonance image. An intensity value will change responsive to a success or failure to successfully perform ablation at the post-ablation location, e.g., as the ablated tissue will scar. Thus, providing information responsive to the intensity value(s) at the post-ablation location provides useful information for a clinician.

In some examples, for each post-ablation location, the one or more magnetic resonance images of the post-ablation location comprise two or more magnetic resonance images of the post-ablation location representing different views of the post-ablation location. The different views are preferably orthogonal views.

The method may further comprise, for each post-ablation location, obtaining electrophysiological information that comprises a value for one or more electrophysiological properties of the post-ablation location, wherein for each post-ablation location, controlling the visual representation of the post-ablation location on the visual representation of the anatomical structure is further responsive to the electrophysiological information of the post-ablation location.

In some examples, for each post-ablation location, the one or more electrophysiological properties comprises one or more of the following: an activation time at the ablation location; an impedance at the ablation location; and/or an ECG trace at the ablation location.

In some examples, for each post-ablation location, the one or more characteristics of the previous ablation comprise one or more of the following: a time at which the previous ablation was performed; a duration of the previous ablation; and/or a temperature of the previous ablation.

Optionally, for each post-ablation location: the previous ablation was provided by supplying an electrical signal to an electrode at the post-ablation location; and the one or more characteristics of the previous ablation comprise one or more of the following: a voltage of the electrical signal; a power of the electrical signal; and/or an energy of the electrical signal over the ablation.

In some examples, the one or more ablations are performed using an ablation catheter; the computer-implemented method further comprises, for each post-ablation location, obtaining tracking information that identifies the location of the ablation catheter during the previous ablation performed at the post-ablation location; and for each post-ablation location, controlling the visual representation of the post-ablation location on the visual representation of the anatomical structure is further responsive to the tracked location of the ablation catheter during the previous ablation performed at the post-ablation location.

In some examples, for each post-ablation location, controlling the visual representation of the post-ablation location on the visual representation of the anatomical structure comprises controlling one or more of visual properties of the visual representation.

In some embodiments for each post-ablation location the one or more visual properties comprises a first visual property and a second visual property; and controlling the visual representation of the post-ablation location on the visual representation of the anatomical structure comprises: controlling the first visual property responsive to the ablation information; and controlling the second visual property responsive to the MRI-derived information.

In some examples, for each post-ablation location: the step of controlling the first visual property is independent of the MRI-derived information; and/or the step of controlling the second visual property is independent of the ablation information.

The one or more visual properties of the visual representation may include one or more of: a size of the visual representation; a shape of the visual representation; a color of the visual representation; an opacity of the visual representation; a blinking frequency and/or pattern of the visual representation; an animation of the visual representation; and/or a pulsing frequency and/or pattern of the visual representation.

There is also proposed a computer program product comprising computer program code which, when executed on a computing device having a processing system, cause the processing system to perform all of the steps of any herein disclosed method.

There is also proposed a processing system for controlling the visual representation of one or more post-ablation locations on a visual representation of an anatomical structure that has undergone one or more ablations.

The processing system is configured to, for each post-ablation location obtain ablation information that comprises a value for each of one or more characteristics of a previous ablation that was performed on the anatomical structure at the post-ablation location; obtain MRI-derived information derived from one or more magnetic resonance images of the post-ablation location; and control the visual representation of the post-ablation location on the visual representation of the anatomical structure responsive to both the ablation information and the MRI-derived information.

There is also proposed a user interface system comprising the processing system; and a user interface, communicatively coupled to the processing system, configured to provide the visual representation of the anatomical structure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an environment in which embodiments can be employed;
Fig. 2 illustrates a proposed method;
Fig. 3 illustrates another proposed method; and
Fig. 4 illustrates a proposed processing system.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs. are merely schematic and are not drawn to scale. It should also be understood that the same reference numerals are used throughout the Figs. to indicate the same or similar parts.

The invention provides a mechanism for controlling a display of an anatomical structure. In particular, one or more visual properties of a post-ablation location on the anatomical structure are controlled responsive to ablation information (about an ablation performed at the post-ablation location) and MRI-derived information derived from one or more MR images obtained after the performance of the ablation.

In the field of ablation therapy or treatment, it is also known to generate a visual representation of the anatomical structure in/on which ablation is to be, or has been, performed by the ablation system. The visual representation of the anatomical structure may be constructed using a (virtual) model of the anatomical structure.

A map or model of the anatomical structure may, for instance, be produced or constructed by monitoring the location of an interventional device within the anatomical structure and producing a map based on the monitored locations, e.g., as disclosed in the European Patent Applications having publication numbers EP 3,558,151 A1 and EP 4,036,930 A1 or using the KODEX-EPD system, such as explained by Tovia Brodie, Oholi, et al. "Anatomical accuracy of the KODEX-EPD novel 3D mapping system of the left atrium during pulmonary vein isolation: A correlation with computer tomography imaging." Journal of Cardiovascular Electrophysiology 33.4 (2022): 618-625.

Alternatively, the model of the anatomical structure may be produced by performing segmentation of one or more images of the anatomical structure, e.g., one or more magnetic resonance images.

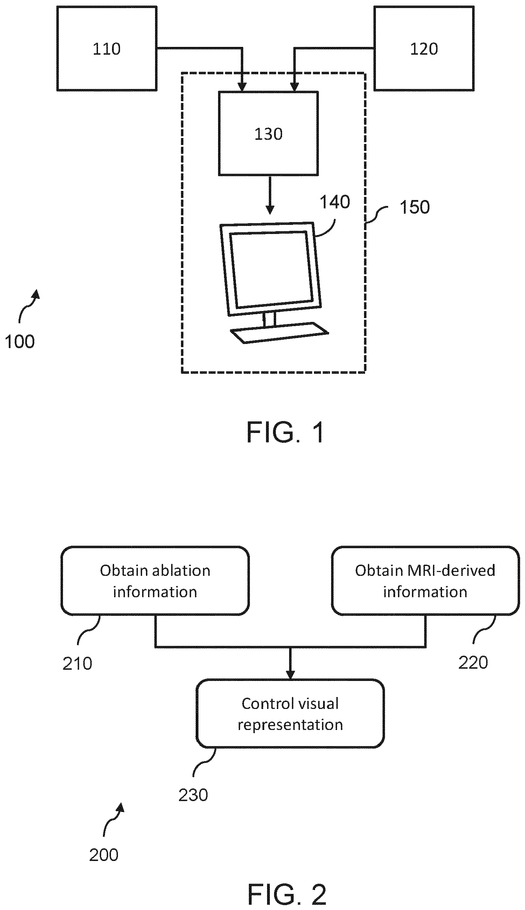

Fig. 1 illustrates an environment 100 in which proposed embodiments may be employed. The environment 100 comprises an ablation system 110, a magnetic resonance imaging (MRI) system 120, a processing system 130 and a user interface 140. The processing system 130 and user interface 140 together define a user interface system 150.

The ablation system 110 is configured to perform ablation on an anatomical structure of a subject, such as the heart, liver or kidney. In particular, the ablation system 110 may comprise one or more ablation catheters (i.e., an interventional ablation device) configured to perform ablation at one or more ablation locations of an anatomical structure.

Example techniques for performing ablation are well established, such as RF ablation, cryoablation, electroporation and/or radiation therapy. Accordingly, the ablation catheter may carry one or more ablation elements for performing ablation, such as an ablation electrode for performing RF ablation.

The ablation catheter(s) is/are sized/configured to be movable within an anatomical cavity (bounded by the anatomical structure) of the subject. For instance, the ablation device may be sized or otherwise configured to be positionable within the heart of the subject.

It is known for an ablation system to track the location or position of the ablation catheter with respect to the anatomical structure and/or the model of the anatomical structure (previously described). The location or position may be defined in a Euclidean space.

Location/position tracking can be performed using any known device tracking technique, particularly those that track a location of a field-sensitive sensor or field-sensitive electrode carried by the ablation catheter by monitoring the response of the sensor to crossing electric and/or magnetic fields. Known approaches are disclosed by: International Patent Application having Publication No. WO 9,404,938 A1 (which makes use of magnetic fields); European Patent Application having Publication No. EP 2,568,880 A1 (which uses fields generated by injecting an electric current); or European Patent Application having Publication No. EP 4,026,494 A1 (which tracks using electric fields).

Other approaches use non-invasive imaging (such as MR imaging) to track the location of the catheter/device. This can be performed, for instance, by employing one or more micro coils located in in the tip of the device and use MR imaging to obtain the location of the device. One known approach is disclosed by US Patent No. 6,246,896 B 1.

The MRI system 120 is configured to generate one or more MR images of the anatomical structure. Example elements, structures, configurations and operations of an MRI system 120 for generating one or more MR images are well known in the art, and are not disclosed for the sake of conciseness. Different types of MR images may be generated and exploited, some examples of which are given later in this disclosure.

In the context of an MRI system for use alongside an ablation system, it is common for the MRI system to generate pre- and post-ablation MR images (i.e., MR images produced from data captured, respectively, before and after ablation has been performed using the ablation system). This advantageously facilitates magnetic resonance based tissue characterization of post-ablation locations. Comparing pre- and post-ablation MR data allows for assessment of the change in tissue caused by the ablation.

The processing system 130 is configured to receive or obtain data produced by the ablation system 110 and/or the MRI system 120 and control the user interface 140 to provide a visual representation of this information.

For instance, the processing system 130 may receive information about a location of an ablation catheter, with respect to the anatomical structure, and control the user interface to provide a visual representation of both the anatomical structure and the relative location of the ablation catheter. This is useful for an operator to visually track the location of the ablation device when performing ablation.

As another example, the processing system 130 may receive the MR image(s) produced by the MRI system and control the user interface 140 to provide a visual representation of the MR image(s), i.e., display the MR images. This is useful for an operator, for instance, to understand the effect and/or success of one or more attempted ablations on the anatomical structure.

Accordingly, the user interface 140 may be configured or controlled, by the processing system, to display or provide a visual representation of characteristics of ablation and/or MR images. Similarly, the user interface 140 may be configured to provide a visual representation of the anatomical structure. Approaches for generating a visual representation of an anatomical structure have been previously disclosed.

The present disclosure provides a mechanism for controlling the visual representation of post-ablation locations on a visual representation of the anatomical structure, e.g., provided by the user interface 140.

As it is possible to track the ablation catheter with respect to the anatomical structure, e.g., using a model thereof, it is possible to identify any location at which ablation is performed or attempted with respect to the anatomical structure (i.e., any "post-ablation location"). Accordingly, it is possible to configure a visual representation of the anatomical structure to identify or indicate post-ablation locations. More specifically, it is possible to configure a visual representation of the anatomical structure to indicate the relative location of post-ablation locations with respect to the anatomical structure.

The present disclosure proposes to control the visual representation of the post-ablation locations to be responsive to at least ablation information and MRI-derived information.

Put another way, the present disclosure proposes to (automatically) enrich post-ablation points with additional information about the ablation as well as data derived from MR data. This will significantly improve a workflow in reviewing the effect of an ablation when performed in an MRI environment. Approaches can be integrated into an MRI suite, e.g., one designed for supporting MRI-guided EP procedures.

The ablation information comprises a value for each of one or more characteristics of a previous ablation that was performed at the ablation location. The one or more characteristics include one or more non-location or non-position characteristics, i.e., characteristics that do not merely represent a (relative) location at which the ablation was performed. Suitable examples are provided later.

The MRI-derived information is information derived from one or more MR images that include the post-ablation location. The one or more MR images may be produced after the ablation is performed, such that they contain information that indicates an outcome of the ablation at the ablation location.

This approach advantageously configures a same visual representation to identify or convey information previously produced by two separate systems. This increases an amount of information that is made available to an operator or viewer of the visual representation.

Fig. 2 illustrates a computer-implemented method 200 for controlling the visual representation of one or more post-ablation locations on a visual representation of an anatomical structure that has undergone one or more ablations. The computer-implemented method 200 may be performed by the processing system previously described.

In particular, the computer-implemented method 200 represents a method performed during at least a first (example) mode of operation of the processing system. Other (optional) modes of operation are later described.

The method 200 comprises a step 210 of obtaining, for each post-ablation location, ablation information that comprises a value for each of one or more characteristics of a previous ablation that was performed on the anatomical structure at the post-ablation location.

Step 210 may be performed by retrieving the ablation information from the ablation system and/or a memory/storage system that stores information produced by the ablation system. The ablation information is produced before the performance of method 200 using the ablation information.

The method 200 also comprises a step 220 of obtaining, for each post-ablation location, MRI-derived information derived from one or more magnetic resonance images of the post-ablation location.

For the first mode of operation, the MRI-derived information may comprise first information about the post-ablation location derived from one or more magnetic resonance images of the post-ablation location generated after the previous ablation was performed on the anatomical structure at the post-ablation location.

In particular, the first information may be derived from one or more magnetic resonance image(s) representing the anatomical structure after the ablation(s) is performed at the post-ablation location.

Step 220 may be performed by retrieving the MRI-derived information from the MRI system and/or a memory/storage system that stores information produced by the MRI system. Alternatively, step 220 may be performed by retrieving the MR image(s) from the MRI system and/or a memory/storage system and processing the retrieved MR image(s) to produce the MRI-derived information. The MR image(s) may be produced before the method 200 is performed.

Step 230 comprises controlling the visual representation of each post-ablation location on the visual representation of the anatomical structure responsive to both the ablation information and the MRI-derived information (of the respective post-ablation location).

Step 230 may, for instance, comprise controlling a user interface to provide a visual representation of the anatomical structure, defining one or more visual properties of the post-ablation location(s) responsive to the ablation information and the MRI-derived information of each post-ablation location.

Thus, for each post-ablation location, step 230 of controlling the visual representation of the post-ablation location on the visual representation of the anatomical structure may comprise controlling one or more of visual properties of the visual representation.

Examples of suitable visual properties of the visual representation include one or more of: a size of the visual representation; a shape of the visual representation; a color of the visual representation; an opacity of the visual representation; a blinking frequency and/or pattern of the visual representation; an animation of the visual representation; and/or a pulsing frequency and/or pattern of the visual representation.

For each post-ablation location, the visual property/properties of the visual representation that are controlled responsive to the ablation information may be different to the visual property of the visual representation. Thus, a first visual property of the visual representation may be responsive to the ablation location and a second (different) visual property may be responsive to the MRI-derived information. Of course, in such an embodiment, the first visual property is independent of the MRI-derived information and the second visual property is independent of the ablation information.

Accordingly, step 230 may comprise (for each post-ablation location) controlling a first visual property of the visual representation of the post-ablation location responsive to the ablation information (and optionally independent of the MRI-derived information); and controlling a second, different visual property of the visual representation of the post-ablation location responsive to the MRI-derived information (and optionally independent of the ablation information).

As previously explained, for the purposes of step 210, the ablation information comprises, for each post-ablation location, one or more characteristics of at least one previous ablation performed at the post-ablation location. The ablation system may record value(s) for each of the one or more characteristics during the ablation, which can be stored (before the performance of method 200) and subsequently retrieved by the processing system (e.g., in step 210).

A number of examples for suitable characteristics of a previous ablation are hereafter described, although the skilled person will appreciate that different characteristics may be employed and may be at least partly dependent upon the type of ablation performed.

The one or more characteristics of the previous ablation may comprise a time at which the previous ablation was performed, e.g., a time since the previous ablation was performed and/or a timestamp at which the previous ablation was performed. The time may indicate a time at which the previous ablation began or a time at which it ended.

The one or more characteristics of the previous ablation may comprise a duration of the previous ablation. The duration may, for instance, be indicated in seconds or milliseconds.

The one or more characteristics of the previous ablation may comprise a temperature of the previous ablation. The temperature may be recorded by a dedicated temperature probe of the ablation system and/or predicted using known control parameters of the ablation system.

In some examples, the previous ablation(s) is/are performed by supplying an electrical signal to an electrode at the post-ablation location. In such embodiments, the one or more characteristics may comprise one or more of the following: a voltage of the electrical signal; a power of the electrical signal; and/or an energy of the electrical signal over the ablation. Such characteristics provide information on the magnitude or intensity of ablation previously performed at the post-ablation location.

In some examples, the previous ablation(s) is/are performed using a cryoablation technique, e.g., by applying or supplying a coolant to the post-ablation location. In such examples, the one or more characteristics may include one or more of the following: number of free-thaw cycles; penetration depth and so on.

For each post-ablation location, the characteristic(s) of the previous ablation that are used, in step 230, to control the visual representation of the post-ablation location may comprise a combination of any one or more of the characteristics previously identified. In some examples, the combination is predetermined. In other examples, the combination is identified or indicated by an operator or user of the user interface, e.g., via a user input.

As previously explained, for the purposes of step 220, the MRI-derived information may comprise, for each post-ablation location, information about the post-ablation location derived from one or more magnetic resonance images of the post-ablation location generated after the previous ablation was performed on the anatomical structure at the post-ablation location.

It is possible to spatially register one or more magnetic resonance images with an (digital) model of an anatomical structure. This can be performed, for instance, using a segmentation technique to identify regions of the imaged subject in the magnetic resonance image and mapping these identified regions to corresponding known regions of the (digital) model of the anatomical model. Other suitable approaches are known in the art.

Once the magnetic resonance image(s) are spatially registered in this way, it is possible to identify areas in the magnetic resonance image(s) that represent post-ablation locations. In particular, as the post-ablation locations are known by tracking the ablation catheter, it is possible to identify a representation of a post-ablation location on the (digital) model and identify the region(s) of the magnetic resonance image(s) that spatially correspond to the post-ablation location.

Other approaches for spatially registering ablation information to MRI-derived information will be apparent to the skilled person. In particular, the MRI-derived information may be pre-registered or automatically registered with the ablation information (e.g., if the ablation catheter is tracked using the MRI system or similar).

The MRI-derived information, for each post-ablation location, may comprise, for each magnetic resonance image of the post-ablation location, at least one intensity value derived from one or more areas representing the post-ablation location within the magnetic resonance image. The MRI-derived information may, for instance, contain a set of one or more values for each post-ablation location. Each value in a same set may represent an average intensity of a different magnetic resonance image at the respective post-ablation location.

The one or more MR images may, for instance, comprise one or more conventional MR images; T1-weighted images; T2-weighted images; T1-rho-weighted, T1-maps; T2-maps; T1-rho-maps, temperature maps; strain maps, diffusion weighted, ADC maps; and/or multidimensional images (e.g., multi-echo or dynamic images). Other MR images are known to the skilled person.

In some examples, the one or more magnetic resonance images of the post-ablation location comprise two or more magnetic resonance images of the post-ablation location representing different views of the post-ablation location.

The MRI-derived information may be generated in advance of method 200, e.g., by the MRI system, and retrieved (e.g., from the MRI system) during step 220. In other examples, the MRI-derived information may be produced in step 220 by processing one or more MR images produced by the MRI system. Thus, step 220 may comprise retrieving the MR image(s) of the post-ablation location(s) and generating MRI-derived information from the retrieved images.

For each post-ablation location, the content of the MRI-derived information used, in step 230, to control the visual representation of the post-ablation location may comprise a set of one or more values derived from a respective different magnetic resonance image - the selection of which may be predetermined and/or identified or indicated by an operator or user of the user interface, e.g., via a user input.

In some examples, the method comprises, for each post-ablation location, obtaining tracking information that identifies the location of the ablation catheter during the previous ablation performed at the post-ablation location; and for each post-ablation location, controlling the visual representation of the post-ablation location on the visual representation of the anatomical structure is responsive to the tracked location of the ablation catheter during the previous ablation performed at the post-ablation location.

It has previously been explained how the previously described method 200 may represent a method performed during a first mode of operation of the processing system. Further modes of operation of the processing system may configure the visual representation of each post-ablation location to be responsive to different combinations of one or more instances of information (about the post-ablation location).

In particular, when the processing system is operating in a particular mode of operation, the visual representation of the post-ablation location may be responsive to one or more of the following instances of information: ablation information; MRI-derived information containing first ("post-ablation") information and/or second ("pre-ablation") information; and/or electrophysiological information containing third ("post-ablation") and/or fourth ("pre-ablation") information.

The previously disclosed first mode of operation controls the visual representation of the post-ablation location to be responsive to ablation information and MRI-derived information containing first ("post-ablation") information (e.g., and no other instances of information as previously listed).

A variation of the first mode of operation controls the visual representation of the post-ablation location to be responsive to ablation information and MRI-derived information containing second ("pre-ablation") information (e.g., and no other instances of information as previously listed). An explanation of second information is provided later in this disclosure.

A wide variety of alternative modes of operation and combinations of instances of information for each post-ablation location will be readily apparent to the skilled person. The mode of operation of the processing system may be responsive to a user input and/or user indication, e.g., provided at a user interface. Thus, the user may be able to select or control to which instances of information the visual representation of the post-ablation information is responsive.

If a visual representation of a post-ablation location is responsive to more than one instance of information, then each instance of information may (independently) define a value for a respective, different visual property of the visual representation of the post-ablation location. In this way, it is possible to visually discriminate between different information about the post-ablation location.

Further examples of alternative modes of operation are hereafter described. The following examples also demonstrate example content for the instances of information previously mentioned.

In a (optional) second mode of operation, the processing system is configured to control the visual representation of each post-ablation location to be responsive to ablation information and MRI-derived information comprising both first ("post-ablation") information and second ("pre-ablation") information (e.g., and no other instances of information as previously listed).

Thus, referring to Fig. 2, the MRI-derived information obtained in step 220 may further comprise second information about the post-ablation location derived from one or more magnetic resonance images of the post-ablation location generated before the previous ablation was performed on the anatomical structure at the post-ablation location. This second information is an example of "pre-ablation information".

Similarly, the step 230 of controlling the visual representation of the post-ablation location is responsive to the ablation information of the post-ablation location and the MRI-derived information of the post-ablation location (which contains both first information and second information).

The second information may differ from the first information only in the timing at which the magnetic resonance image(s) are originally captured, i.e., the time at which the raw data for producing the magnetic resonance image(s) is originally produced (i.e., the corresponding MRI scan is performed). In particular, the second information may be derived from one or more magnetic resonance image(s) representing the anatomical structure before the ablation(s) is performed at the post-ablation location. By contrast, the first information may be derived from one or more magnetic resonance image(s) representing the anatomical structure after the ablation(s) is performed at the post-ablation location.

The second mode of operation facilitates ease of comparison between pre- and post-ablation status of the post-ablation location. This allows for increased ease and efficiency of understanding the effect and/or success of an attempted ablation of a target location on the anatomical structure.

In some examples, for each post-ablation location, the ablation information, first information (of the MRI-derived information) and second information (of the MRI-derived information) define one or more values for different visual properties (e.g., size, color, shape).

As a working example, step 230 may comprise controlling: the color of the visual representation of the post-ablation location to be responsive to the ablation information (of the post-ablation location); the size of the visual representation of the post-ablation location to be responsive to the first information of the MRI-derived information (of the post-ablation location); and the shape of the visual representation of the post-ablation location to be responsive to the second information of the MRI-derived information (of the post-ablation location).

In some examples, for each post-ablation location, a same visual property (e.g., size, color, shape etc.) of the visual representation is responsive to the first information and the second information of the MRI-derived information. In particular, the visual representation may alternate between a first value and a second value for the same visual property, where the first value is responsive to the first information of the MRI-derived information of the post-ablation location and the second value is responsive to the first information of the MRI-derived information of the post-ablation location. Such an approach facilitates each of recognizing or identifying the effect of the ablation performed at the post-ablation location.

As a working example, step 230 may comprise controlling the color of the visual representation of the post-ablation location to be responsive to the ablation information (of the post-ablation location). Step 230 may comprise controlling the size of visual representation to alternative between a first size and a second size. The first size is responsive to the first information of the MRI-derived information of the post-ablation location. The second size is responsive to the second information of the MRI-derived information of the post-ablation location.

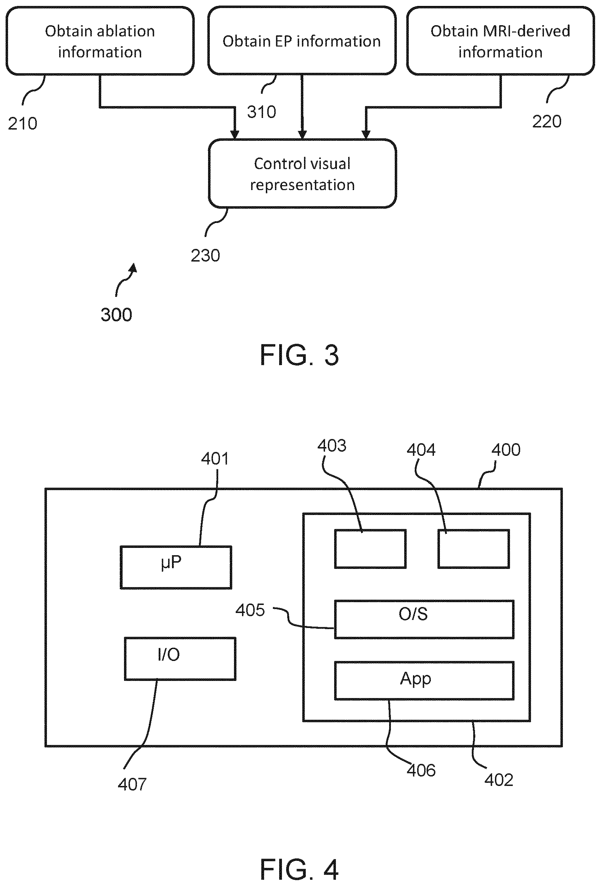

Fig. 3 illustrates another computer-implemented method 300 for controlling the visual representation of one or more post-ablation locations on a visual representation of an anatomical structure that has undergone one or more ablations. The computer-implemented method 300 may be performed by the processing system previously described.

In particular, the computer-implemented method 300 represents a method performed during at least a third and/or fourth (example) mode of operation of the processing system.

The method 300 differs from the previously described method (described with reference to Fig. 2) by further comprising a step 310 of obtaining electrophysiological information that comprises a value for one or more electrophysiological properties of the post-ablation location. Correspondingly, step 230 is modified such that the visual representation of the post-ablation location on the visual representation of the anatomical structure is further responsive to the electrophysiological information of the post-ablation location.

The one or more electrophysiological properties may comprise one or more of the following: an activation time at the ablation location; an impedance at the ablation location; and/or an ECG trace at the ablation location. Other examples of electrophysiological properties are well known to the skilled person.

Approaches for determining a value for each identified electrophysiological property are well known in the art. For instance, a value for one or more electrical properties of one or more electrodes of the ablation catheter (or other interventional device) may be monitored in order to determine the value.

In a third mode of operation, for each post-ablation location, the electrophysiological information comprises third information that comprises a value for one or more electrophysiological properties of the post-ablation location derived from data gathered after the previous ablation was performed on the anatomical structure at the post-ablation location. Thus, the third information may represent post-ablation electrophysiological information.

Thus, in the third mode of operation, the step 230 of controlling the visual representation of the post-ablation location is responsive to the ablation information of the post-ablation location, the MRI-derived information of the post-ablation location and the electrophysiological information that comprises third information of the post-ablation location.

In a fourth mode of operation, for each post-ablation location, the electrophysiological information comprises fourth information that comprises a value for one or more electrophysiological properties of the post-ablation location derived from data gathered before the previous ablation was performed on the anatomical structure at the post-ablation location. Thus, the fourth information may represent pre-ablation electrophysiological information.

Thus, in the fourth mode of operation, the step 230 of controlling the visual representation of the post-ablation location is responsive to the ablation information of the post-ablation location, the MRI-derived information of the post-ablation location and the electrophysiological information that comprises fourth information of the post-ablation location.

In a fifth mode of operation, for each post-ablation location, the electrophysiological information comprises the third information and the fourth information. Thus, in the fifth mode of operation, the step 230 of controlling the visual representation of the post-ablation location is responsive to the ablation information of the post-ablation location, the MRI-derived information of the post-ablation location and the electrophysiological information that comprises third information and fourth information of the post-ablation location.

In some examples, when operating in the third mode of operation, the step 230 of controlling the visual representation of the post-ablation location is independent of any fourth information. Similarly, in some examples, when operating in the fourth mode of operation, the step 230 of controlling the visual representation of the post-ablation location is independent of any third information.

Fig. 4 illustrates an example of a processing system 400 within which one or more parts of an embodiment may be employed. The processing system 400 may be integrated as part of the environment previously illustrated in Fig. 1.

In particular, with further reference to Fig. 1, the processing system 400 may function as the processing system 130 of the environment 100. In some examples, the processing system may form, together with the user interface 140, a herein proposed user interface system 150.

Various operations discussed above may utilize the capabilities of the processing system 400. For example, one or more parts of a system for controlling the visual representation of one or more post-ablation locations on a visual representation of an anatomical structure that has undergone one or more ablations may be incorporated in any element, module, application, and/or component discussed herein. In this regard, it is to be understood that system functional blocks can run on a single processing system or may be distributed over several processing systems and locations (e.g., connected via internet).

The processing system 400 includes, but is not limited to, PCs, workstations, laptops, PDAs, palm devices, servers, storages, and the like. Generally, in terms of hardware architecture, the processing system 400 may include one or more processors 401, memory 402, and an input/output interface 407 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 401 is a hardware device for executing software that can be stored in the memory 402. The processor 401 can be virtually any custom made or commercially available processor, a central processing unit (CPU), a digital signal processor (DSP), or an auxiliary processor among several processors associated with the processing system 400, and the processor 401 may be a semiconductor-based microprocessor (in the form of a microchip) or a microprocessor.

The memory 402 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 402 may incorporate electronic, magnetic, optical, and/or other types of storage media. Note that the memory 402 can have a distributed architecture, where various components are situated remote from one another, but can be accessed by the processor 401.

The software in the memory 402 may include one or more separate programs, each of which comprises an ordered listing of executable instructions for implementing logical functions. The software in the memory 402 includes a suitable operating system (O/S) 405, compiler 404, source code 403, and one or more applications 406 in accordance with exemplary embodiments. As illustrated, the application 406 comprises numerous functional components for implementing the features and operations of the exemplary embodiments. The application 406 of the processing system 400 may represent various applications, computational units, logic, functional units, processes, operations, virtual entities, and/or modules in accordance with exemplary embodiments, but the application 406 is not meant to be a limitation.

The operating system 405 controls the execution of other computer programs, and provides scheduling, input-output control, file and data management, memory management, and communication control and related services. It is contemplated by the inventors that the application 406 for implementing exemplary embodiments may be applicable on all commercially available operating systems.

Application 406 may be a source program, executable program (object code), script, or any other entity comprising a set of instructions to be performed. When a source program, then the program is usually translated via a compiler (such as the compiler 404), assembler, interpreter, or the like, which may or may not be included within the memory 402, so as to operate properly in connection with the O/S 405. Furthermore, the application 406 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Python, Perl, Java, ADA, .NET, and the like.

The input/output interface 407 is configured to communicate with a user interface for controlling a visual representation provided at the user interface. The user interface may therefore comprise one or more output devices, for example but not limited to a printer, display, etc. The user interface may further comprise one or more input devices for providing a user input for controlling or defining the operation of the processing system, e.g., to define a mode of operation of the processing system.

The input/output interface 407 may also be configured to communicate with other devices, elements or features, such as the ablation system, the MRI system and/or a storage system that stores data produced by the ablation and/or MRI system.

If the processing system 400 is a PC, workstation, intelligent device or the like, the software in the memory 402 may further include a basic input output system (BIOS) (omitted for simplicity). The BIOS is a set of essential software routines that initialize and test hardware at startup, start the O/S 405, and support the transfer of data among the hardware devices. The BIOS is stored in some type of read-only-memory, such as ROM, PROM, EPROM, EEPROM or the like, so that the BIOS can be executed when the processing system 400 is activated.

When the processing system 400 is in operation, the processor 401 is configured to execute software stored within the memory 402, to communicate data to and from the memory 402, and to generally control operations of the processing system 400 pursuant to the software. The application 406 and the O/S 405 are read, in whole or in part, by the processor 401, perhaps buffered within the processor 401, and then executed.

When the application 406 is implemented in software it should be noted that the application 406 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 406 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

It will be understood that disclosed methods are preferably computer-implemented methods. As such, there is also proposed the concept of a computer program comprising code for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figs. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200, 300) for controlling the visual representation of one or more post-ablation locations on a visual representation of an anatomical structure that has undergone one or more ablations, the computer-implemented method comprising, for each post-ablation location:
obtaining ablation information (210) that comprises a value for each of one or more characteristics of a previous ablation that was performed on the anatomical structure at the post-ablation location;
obtaining MRI-derived information (220) derived from one or more magnetic resonance images of the post-ablation location; and
controlling (230) the visual representation of the post-ablation location on the visual representation of the anatomical structure responsive to both the ablation information and the MRI-derived information.

2. The computer-implemented method (200, 300) of claim 1, wherein, for each post-ablation location, the MRI-derived information comprises, for each magnetic resonance image of the post-ablation location, at least one intensity value derived from one or more areas representing the post-ablation location within the magnetic resonance image.

3. The computer-implemented method (200, 300) of claim 1 or 2, wherein, for each post-ablation location, the one or more magnetic resonance images of the post-ablation location comprise two or more magnetic resonance images of the post-ablation location representing different views of the post-ablation location.

4. The computer-implemented method (300) of any one of claims 1 to 3, further comprising, for each post-ablation location, obtaining (310) electrophysiological information that comprises a value for one or more electrophysiological properties of the post-ablation location,
wherein for each post-ablation location, controlling (230) the visual representation of the post-ablation location on the visual representation of the anatomical structure is further responsive to the electrophysiological information of the post-ablation location.

5. The computer-implemented method (300) of claim 4, wherein, for each post-ablation location, the one or more electrophysiological properties comprises one or more of the following: an activation time at the ablation location; an impedance at the ablation location; and/or an ECG trace at the ablation location.

6. The computer-implemented method (200, 300) of any one of claims 1 to 5, wherein, for each post-ablation location, the one or more characteristics of the previous ablation comprise one or more of the following: a time at which the previous ablation was performed; a duration of the previous ablation; and/or a temperature of the previous ablation.

7. The computer-implemented method (200, 300) of any one of claims 1 to 6, wherein, for each post-ablation location:
the previous ablation was provided by supplying an electrical signal to an electrode at the post-ablation location; and
the one or more characteristics of the previous ablation comprise one or more of the following: a voltage of the electrical signal; a power of the electrical signal; and/or an energy of the electrical signal over the ablation.

8. The computer-implemented method (200, 300) of any one of claims 1 to 7, wherein:
the one or more ablations are performed using an ablation catheter;
the computer-implemented method further comprises, for each post-ablation location, obtaining tracking information that identifies the location of the ablation catheter during the previous ablation performed at the post-ablation location; and
for each post-ablation location, controlling (230) the visual representation of the post-ablation location on the visual representation of the anatomical structure is responsive to the tracked location of the ablation catheter during the previous ablation performed at the post-ablation location.

9. The computer-implemented method (200, 300) of any one of claims 1 to 8, wherein, for each post-ablation location, controlling (230) the visual representation of the post-ablation location on the visual representation of the anatomical structure comprises controlling one or more of visual properties of the visual representation.

10. The computer-implemented method (200, 300) of claim 9, wherein, for each post-ablation location:
the one or more visual properties comprises a first visual property and a second visual property; and
controlling (230) the visual representation of the post-ablation location on the visual representation of the anatomical structure comprises:
controlling the first visual property responsive to the ablation information; and
controlling the second visual property responsive to the MRI-derived information.

11. The computer-implemented method (200, 300) of claim 10, wherein, for each post-ablation location:
the step of controlling the first visual property is independent of the MRI-derived information; and/or
the step of controlling the second visual property is independent of the ablation information.

12. The computer-implemented method (200, 300) of any one of claims 9 to 11, wherein the one or more visual properties of the visual representation include one or more of: a size of the visual representation; a shape of the visual representation; a color of the visual representation; an opacity of the visual representation; a blinking frequency and/or pattern of the visual representation; an animation of the visual representation; and/or a pulsing frequency and/or pattern of the visual representation.

13. A computer program product comprising computer program code which, when executed on a computing device having a processing system (130, 400), cause the processing system to perform all of the steps of the method according to any one of claims 1 to 12.

14. A processing system (130, 400) for controlling the visual representation of one or more post-ablation locations on a visual representation of an anatomical structure that has undergone one or more ablations, the processing system being configured to, for each post-ablation location:
obtain (210) ablation information that comprises a value for each of one or more characteristics of a previous ablation that was performed on the anatomical structure at the post-ablation location;
obtain (220) MRI-derived information derived from one or more magnetic resonance images of the post-ablation location; and
control (230) the visual representation of the post-ablation location on the visual representation of the anatomical structure responsive to both the ablation information and the MRI-derived information.

15. A user interface system (150) comprising:
the processing system (130, 400) of claim 14; and
a user interface (140), communicatively coupled to the processing system, configured to provide the visual representation of the anatomical structure.
